# EUROPEAN PATENT SPECIFICATION

(11) **EP 4 408 399 B1**
(45) Date of publication and mention of the grant of the patent: **14.05.2025**
(21) Application number: 22790053.7
(22) Date of filing: 30.09.2022
(51) Int. Cl.: A61K 9/107, A61P 3/10, A61K 47/44, A61K 31/58

(54) **A NANO EMULSIFIED PHYTO-DRUG FOR TRANSDERMAL TREATMENT OF DIABETES**
NANOEMULGIERTES PHYTOARZNEIMITTEL ZUR TRANSDERMALEN BEHANDLUNG VON DIABETES
PHYTOMÉDICAMENT NANO-ÉMULSIFIÉ POUR LE TRAITEMENT TRANSDERMIQUE DU DIABÈTE

(30) Priority: 01.10.2021 ZA 202107408
(43) Date of publication of application: 07.08.2024
(73) Proprietor: University of South Africa, 0001 Pretoria (ZA)
(72) Inventor: AKINSIPO, Oyesolape, Basirat, Ijebu Ode Ogun State (NG); DARE, Enock, Olugbenga, Abeokuta Ogun State (NG); ALAYANDE, Samson, Oluwagbemiga, Ibadan (NG); OLADOYINBO, Fatai, Oladipupo, Ogun State (NG); SANNI, Lateef, Ogun State (NG); KATARE, Deepshikha, Pande, Noida 201303, Uttar Pradesh (IN); MSAGATI, Titus, Alfred, Makudali, 1709 Johannesburg (ZA)
(74) Representative: J A Kemp LLP
(86) International application number: PCT/IB2022/059329
(87) International publication number: WO 2023/053077

(56) References cited:
- EP-A1- 1 092 422
- US-A1- 2010 143 509
- SANGEETHA M K ET AL: "PPAR's and Diosgenin a chemico biological insight in N", CHEMICO-BIOLOGICAL INTERACTIONS, ELSEVIER SCIENCE IRLAND, IR, vol. 206, no. 2, 31 August 2013 (2013-08-31), pages 403 - 410, XP028779115, ISSN: 0009-2797, DOI: 10.1016/J.CBI.2013.08.014

## Description

THIS INVENTION relates to transdermal treatment of diabetes. It relates in particular to a formulation for the transdermal delivery of the phytoactive antidiabetic agent diosgenin that is encapsulated and protected by an oil-in-water nanoemulsion matrix for the treatment of Type II Diabetes Mellitus.

Type II Diabetes Mellitus is the most common type of diabetes worldwide. It is associated with increased blood glucose and contributes to a high rate of cardiovascular morbidity and mortality. While the cornerstone therapy to treating Type II Diabetes is weight loss, regular exercise and diet, antidiabetic drugs are used to maintain blood sugar. Type II Diabetes Mellitus is treated with oral hypoglycemics (sulfonylureas, biguanides, α-glucosidase inhibitors, meglitinide analogues and thiazolidinediones). The conventional dosage forms of oral hypoglycemics are challenged due to the need for frequent dosing, shortened half live and low bioavailability. There are significant complications and side effects posed by parenteral drugs or oral route of delivery, including invasive drug delivery systems; gastrointestinal bowel irritation due to interaction of the drug with other intestinal substances; drug reduction due to extensive first pass metabolism and toxicity; toxicity and poor aqueous solubility.

Advances have been made to improve drug delivery and to alleviate these challenges. These advances include drug delivery systems that deliver drugs at a constant rate and sustained release, reduced side effects and frequent doses. These drug delivery systems include microspheres, nanosystems, hydrogels, transdermal delivery systems, niosomes and controlled release tablets. Patent document EP1092422 discloses oil-in-water nanoemulsions comprising diosgenin suitable for transdermal delivery of diosgenin to a patient to treat type II diabetes mellitus.

The invention addresses these problems arising from the use of parenteral drugs or oral route of delivery by providing a non-invasive and naturally formulated transdermal delivery system for a phytoactive antidiabetic agent - diosgenin. Diosgenin is a natural steroidal sapogenin that is extracted from herbs including *Dioscorea rhizome, Dioscorea villosa, Trigonella foenum-graecum, Smilax China,* and *Rhizoma polgonati.* It is used in the treatment of various diseases and medical conditions including cancers, hyperlipidemia, inflammation, infections and diabetes. In the treatment of diabetes, diosgenin has been shown to be effective in regulating the targets and pathways of glycolipid metabolism, apoptosis, inflammation and oxidative stress. To ensure targeted and sustained delivery of diosgenin, the invention discloses a formulation including a nanoemulsion matrix that engulfs and protects diosgenin until it systemically reaches its target.

The invention discloses a nanoemulsified formulation according to the claims that comprises:
- An oil phase, in particular a mixture of sesame oil and bottle gourd seed oil extracted from the seeds of bottle gourd (*Lagenaria sphaerica)* as the oil phase of the formulation.
- Emulsifiers: non-ionic surfactants (Tween 80) and co-surfactants (glycerol) that are non-toxic and enhance permeation.
- Water as the required dispersing aqueous medium for emulsion formation to facilitate transdermal delivery.
- Diosgenin as the therapeutic agent.

The formulation is therefore a naturally formulated nanoemulsified system. This allows it to permeate the skin to deeply penetrate and cross all barriers to enter the systemic circulation in a controlled manner. The nanoemulsion is prepared using low energy and ambient temperature which is beneficial for its cost effectiveness, ease of accessibility and ability to protect the efficacy of a drug throughout the preparation process by preventing the decomposition of thermolabile active ingredients since little or no temperature is required and instead, it is shown that it is sufficient to use a simple magnetic stirring.

The formulation allows for ease of administration and patient accessibility. It is a non-invasive mode of delivering diosgenin and is pain-free to the patient. It is shown that the solubility of diosgenin is enhanced when dissolved in combination with bottle gourd seed. Nanoemulsion based carriers are the most suitable delivery systems for poorly soluble drugs by improving the drug's solubility, improving permeation of drugs and ultimately increasing bioavailability by transdermal therapeutic systems.

The formulation can be used to treat diabetes, including Type I diabetes, Type II diabetes, gestational diabetes, maturity onset diabetes, neonatal diabetes, Wolfram syndrome, Alström syndrome and latent autoimmune diabetes. It particular, it is used to treat Type II Diabetes Mellitus by either producing the formulation as a cosmetic or gel for transdermal application, or by producing the formulation as a transdermal patch.

According to a first aspect of the invention there is provided a formulation according to the claims comprising an oil-in-water nanoemulsion matrix encapsulating diosgenin which may be dissolved in an oil phase.

The oil phase may comprise an oil selected from at least one of sesame oil, bottle gourd seed oil, flaxseed oil, omega-3 polyunsaturated fish oil, omega-6 polyunsaturated fish oil, safflower oil, olive oil, pine nut oil, cherry kernel oil, soybean oil, pumpkin oil, pomegranate oil, primrose oil, or a combination thereof.

According to the claims, the oil phase comprises a 1:1 mixture of sesame oil and bottle gourd seed oil *(Lagenaria sphaerica*).

The nanoemulsion matrix may include a non-ionic surfactant and a co-surfactant as emulsifiers.

The non-ionic surfactant may be Tween 80 and the co-surfactant may be glycerol.

The ratio of the oil to water is 4:1.

The formulation may be in the form of a cosmetic, gel, transdermal patch, or similar.

According to a second aspect of the invention there is provided a method according to the claims of producing a formulation comprising a nanoemulsion matrix surrounding diosgenin, wherein the method includes the steps of:
- preparing an oil to form an oil phase;
- adding 15 % emulsifiers in the form of a mixture of 1:1 non-ionic surfactant and a co-surfactant to 60 % of the oil phase;
- dissolving diosgenin in the oil phase to form a diosgenin-in-oil mixture; and
- adding distilled water as a dispersing aqueous medium to the mixture to obtain an oil-in-water nanoemulsion with a 25 % water content.

The oil phase may comprise an oil selected from at least one of sesame oil, bottle gourd seed oil, flaxseed oil, omega-3 polyunsaturated fish oil, omega-6 polyunsaturated fish oil, safflower oil, olive oil, pine nut oil, cherry kernel oil, soybean oil, pumpkin oil, pomegranate oil, primrose oil, or a combination thereof.

The oil phase is prepared by combining sesame oil and bottle gourd seed oil in a 1:1 ratio.

The non-ionic surfactant may be Tween 80 and the co-surfactant may be glycerol.

The ratio of oil to water is 4:1.

The nanoemulsion matrix is prepared under low energy using a magnetic stirrer and ambient temperature.

The distilled water is added dropwise to the mixture while stirring by the magnetic stirrer.

The nanoemulsion matrix may be formed into a nanoemulsified gel. Disclosed, but not claimed, is a formulation comprising a nanoemulsion matrix and diosgenin for use in a method of treating diabetes in a patient, wherein the formulation is for transdermal delivery of diosgenin.

The diabetes may be selected from Type I diabetes, Type II diabetes, gestational diabetes, maturity onset diabetes, neonatal diabetes, Wolfram syndrome, Alström syndrome and latent autoimmune diabetes. It particular, the diabetes may be Type II diabetes mellitus. The formulation may be produced according to the method of the second aspect of the invention.

Disclosed, but not claimed, is a use of diosgenin and a nanoemulsion matrix in the manufacture of a formulation for treatment of diabetes in a patient, wherein the formulation is for transdermal delivery of diosgenin.

The diabetes may be any one of Type I diabetes, Type II diabetes, gestational diabetes, maturity onset diabetes, neonatal diabetes, Wolfram syndrome, Alström syndrome and latent autoimmune diabetes. The diabetes may be Type II diabetes mellitus.

The formulation may be produced according to the method of the second aspect of the invention.

The invention will now be described in more detail with reference to the Example hereunder, and the accompanying drawings.

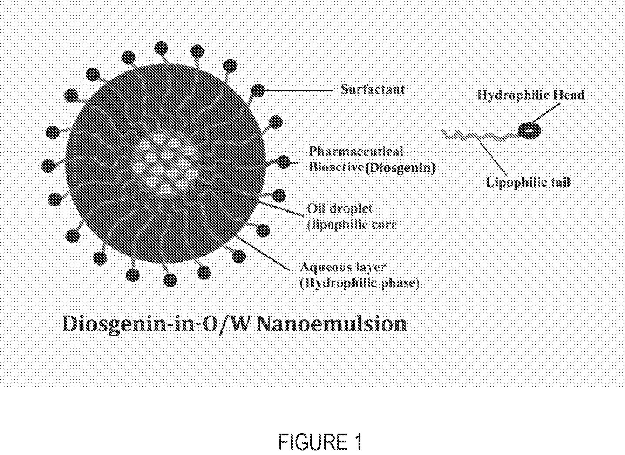

FIGURE 1 shows for the Example, diosgenin in an oil-in-water nanoemulsion;

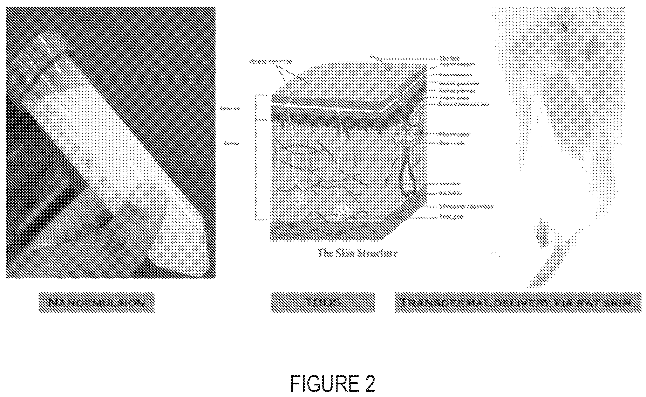

FIGURE 2 shows, for the Example, a representation of the uniquely developed diosgenin-in-oil in water nanoemulsion delivered transdermally through the skin of the diabetic rat;
FIGURE 3 shows, for the Example, therapeutic effect of transdermal diosgenin-in-Nanoemulsion (DGNe/G) on the blood glucose concentration of diabetic rats; and
FIGURE 4 shows, for the Example, effect of diosgenin-in-nanoemulsion (DGNe) transdermal treatment on the HbA1c level of diabetic rats.

### EXAMPLE

### Materials and Methods

In preparing the formulation, the concentration of diosgenin was measured based on the weight of rats. In view of its hydrophobic nature which limits its solubility in water, diosgenin was incorporated in the oil phase before the emulsification process.

### Production of formulation

A mixture of 1:1 sesame oil and bottle gourd seed oil (*Lagenaria sphaerica*) was prepared as the oil phase. Other oils may be used, such as flaxseed oil, omega-3 polyunsaturated fish oil, omega-6 polyunsaturated fish oil, safflower oil, olive oil, pine nut oil, cherry kernel oil, soybean oil, pumpkin oil, pomegranate oil, primrose oil, or a combination thereof.

15 % of an equal mixture of Tween 80 and glycerol was added to 60% of the oil phase.

The formulation was produced through a low energy-phase inversion composition technique with less shearing energy. The shearing procedure was conducted at an ambient temperature all through the preparation.

40 mg/kg body weight (bw) diosgenin was dissolved in the oil phase and stirred at 350 rpm using the magnetic stirring hot plate.

Distilled water was titrated dropwise into the oil phase mixture until a phase inversion into an oil-in-water nanoemulsion was obtained with a 25 % water content.

Optimized nanoemulsion was modified into a nanoemulsified gel for improved skin applicability.

### Preparation of rats for testing

Prior to treatment, albino Wistar rats were treated with streptozotocin (STZ) to induce Type II Diabetes Mellitus (T2DM). The development and progression of the rats into a diabetic state was confirmed by spectrometrically measuring the random blood glucose level of rats at specific intervals. Rats with a sustained blood glucose level beyond 300mg/dL and an HbA1c level ≥ 6.5% were considered hyperglycemic/diabetic.

### Treatment of hyperglycemic/diabetic rats

Diabetic rats were prepared for transdermal treatment with the formulated diosgenin-in-nanoemulsion. 1.11 g of 20 mg/kg body weight (bw) diosgenin was applied at every single application 12 hourly for a period of three weeks. The therapeutic potential of diosgenin against T2DM was determined by measuring the blood glucose concentration (mg/dL) and HbA1c (%) of treated rats at specific intervals.

### Results

Effect of streptozotocin on the blood glucose concentration, glycosylated hemoglobin and HbA1c level in albino Wistar rats.

Table 1 presents the results of how the metabolism of normal albino rats progresses into a hyperglycemic/diabetic state as a multiple low-dosage of streptozotocin is administered for diabetes induction.

**Table 1**

| **S/No** | **Group** | **Glucose Concentration (mg/dL)** | **Glycated Hemoglobin** | | |
|---|---|---|---|---|---|
| | | | GHb | MBG | HbA1c |
| **1.** | Normal Control | 106.59 ± 3.48 | 6.98 ± 0.052 | 123.14 ± 0.637 | 5.903 ± 0.02 |
| | First Cycle | | | | |
| **2.** | Dose 1 | 171.56 ± 22.70 | - | | |
| **3.** | Dose 2 | 318.31 ± 19.86*** | - | | |
| **4.** | Dose 3 | 439.31 ± 41.00*** | - | | |
| **5.** | Day 15 | 532.17 ± 22.15*** | 12.37 ± 0.81 | 225.39 ± 15.55 | 9.50 ± 0.54 |
| | Second cycle | | | | |
| **6.** | Dose 4 | 425.60 ± 31.10*** | | | |
| **7.** | Dose 5 | 459.87 ± 33.56*** | | | |
| **8.** | Dose 6 | 451.89 ± 44.27*** | 12.40 ± 0.28 | 226.03 ± 5.53 | 9.50 ± 0.19 |

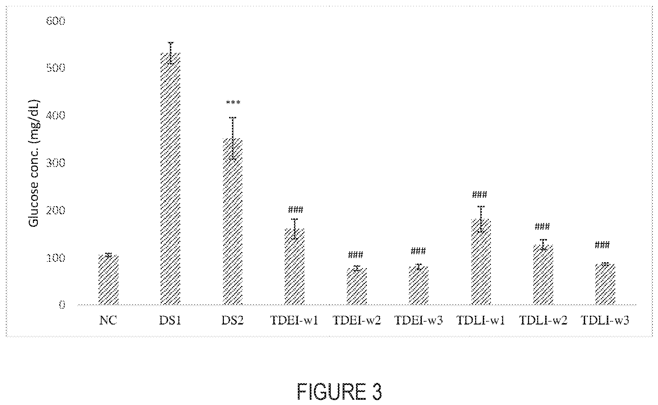

In **Figure 3****,** the significance of the blood glucose concentration is indicated as * p<0.05, ** p<0.01 and ***p<0.001 when normal control, NC (n=5), is compared to disease group, DS (n=20) at stage 1 and 2 (DS-1 and DS-2). ^{#} p<0.05, ^{##} p<0.01 and ^{###}p<0.001 when DS-1 and DS-2 is compared with early intervention and late intervention transdermal treatment (TDEI and TDLI) for three weeks.

In **Figure 3****,** it is evident that the blood glucose concentration in diabetic rats (DS-1 and DS-2) was highly elevated in comparison to the normal control (NC). With a single week's treatment of the rats using the formulation, the blood glucose concentration decreased significantly, with better results seen at early invention compared to late intervention. The blood glucose concentration for both early and late intervention cases decreased in week 2. In the early intervention cases, there was not a significant decrease in blood glucose concentration in week 3 of treatment, but there was a noticeable difference in the late intervention cases in week 3.

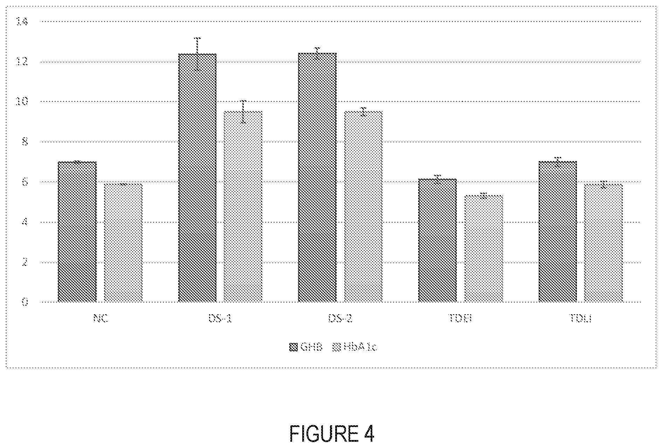

In **Figure 4****,** HbA1c levels were measured at the end of disease induction and at the end of the 3 week treatment. The significance of each measurement is indicated as * p<0.05, ** p<0.01 and ***p<0.001 when normal control (NC) is compared to compared to disease stage 1 and 2 (DS-1 and DS-2). ^{#} p<0.05, ^{##} p<0.01 and ^{###}ρ<0.001 when DS-1 and DS-2 is compared with early intervention and late intervention transdermal treatment (TDEI and TDLI).

In **Figure 4****,** HbA1c (hemoglobin A1c) levels are highly elevated in diabetic rats (DS-1 and DS-2) compared to the normal control. With treatment of the rats using the formulation, HbA1c levels decreased significantly, with better results seen at an early invention compared to late intervention. The HbA1c levels in the early intervention cases decreased to below that of the normal control, and in the late intervention cases the HbA1c levels decreased to the same level as the normal control.

### Discussion

A novel formulation has been developed that uses natural extracts to produce an oil-in-water nanoemulsion matrix that engulfs and protects the drug diosgenin in order to ensure bioavailability, safety, targeted and sustained delivery of the drug to its target. The nanoemulsion acts as a solubilizing, non-invasive protective drug carrier and permeating agent that is administered transdermally to treat diabetes, including Type I diabetes, Type II diabetes, gestational diabetes, maturity onset diabetes, neonatal diabetes, Wolfram syndrome, Alström syndrome, latent autoimmune diabetes, and in particular to treat Type II Diabetes Mellitus.

## Claims

1. A formulation for transdermal delivery of diosgenin to a patient to treat type II diabetes mellitus in the patient, the formulation comprising diosgenin encapsulated in a nanoemulsion matrix, wherein
the nanoemulsion matrix is an oil-in-water nanoemulsion comprising diosgenin dissolved in an oil phase of the nanoemulsion matrix;
the oil phase comprises a 1:1 combination of sesame oil and bottle gourd seed oil; and
the ratio of oil to water is 4:1.

2. The formulation according to claim 1, wherein the nanoemulsion matrix includes a non-ionic surfactant and a co-surfactant.

3. The formulation according to any one of claims 1 to 2, wherein the formulation is in the form of a cosmetic, gel or transdermal patch.

4. A method of producing a formulation comprising a nanoemulsion matrix encapsulating diosgenin for transdermal delivery of diosgenin to a patient to treat type II diabetes mellitus in the patient, wherein the method comprises:
- preparing an oil phase comprising a 1:1 combination of sesame oil and bottle gourd seed oil;
- adding 15 % emulsifiers in the form of a mixture of 1:1 non-ionic surfactant and a co-surfactant to 60 % of the oil phase;
- dissolving 40 mg/kg diosgenin in the oil phase at ambient temperature using a magnetic stirrer; and
- adding distilled water dropwise to the mixture while stirring by the magnetic stirrer to obtain an oil-in-water nanoemulsion with an oil to water ratio of 4:1.

## Patentansprüche

1. Formulierung für eine transdermale Abgabe von Diosgenin an einen Patienten, um einen Diabetes mellitus, Typ II in dem Patienten zu behandeln, wobei die Formulierung Diosgenin umfasst, das in einer Nanoemulsionsmatrix verkapselt ist, wobei
die Nanoemulsionsmatrix eine Öl-in-Wasser-Nanoemulsion ist, die Diosgenin umfasst, das in einer Ölphase der Nanoemulsionsmatrix aufgelöst ist;
die Ölphase eine 1 : 1-Kombination aus Sesamöl und Flaschenkürbissamenöl umfasst; und
das Verhältnis von Öl zu Wasser 4 : 1 beträgt.

2. Formulierung nach Anspruch 1, wobei die Nanoemulsionsmatrix ein nichtionisches Tensid und ein Co-Tensid umfasst.

3. Formulierung nach einem der Ansprüche 1 bis 2, wobei die Formulierung in der Form einer Kosmetik, einem Gel oder einem transdermalen Pflaster vorliegt.

4. Verfahren zum Herstellen einer Formulierung, die eine Nanoemulsionsmatrix umfasst, die Diosgenin einkapselt, für eine transdermale Abgabe von Diosgenin an einen Patienten, um einen Diabetes mellitus, Typ II in dem Patienten zu behandeln, wobei das Verfahren umfasst:
- Herstellen einer Ölphase, die eine 1 : 1-Kombination aus Sesamöl und Flaschenkürbissamenöl umfasst;
- Zugeben von 15 % Emulgatoren in der Form einer Mischung von 1 : 1 nichtionischem Tensid und einem Co-Tensid zu 60 % der Ölphase;
- Auflösen von 40 mg/kg Diosgenin in der Ölphase bei Umgebungstemperatur unter Verwendung eines magnetischen Rührers; und
- tropfenweises Zugeben von destilliertem Wasser zu der Mischung, während sie durch den magnetischen Rührer gerührt wird, um eine Öl-in-Wasser-Nanoemulsion mit einem Verhältnis von Öl zu Wasser von 4 : 1 zu erhalten.

## Revendications

1. Préparation pour administration transdermique de diosgénine à un patient pour traiter le diabète de type II chez le patient, la préparation comprenant de la diosgénine encapsulée dans une matrice de nano-émulsion, dans laquelle
la matrice de nano-émulsion est une nano-émulsion huile-en-eau comprenant de la diosgénine dissoute dans une phase huileuse de la matrice de nano-émulsion ;
la phase huileuse comprend une combinaison 1 : 1 d'huile de sésame et d'huile de graines de courge ; et
le rapport huile-eau est 4 : 1.

2. Préparation selon la revendication 1, dans laquelle la matrice de nano-émulsion inclut un surfactant non ionique et un co-surfactant.

3. Préparation selon l'une quelconque des revendications 1 et 2, dans laquelle la préparation est sous la forme d'un gel cosmétique, d'un gel, ou d'un timbre transdermique.

4. Procédé de production d'une préparation comprenant une matrice de nano-émulsion encapsulant de la diosgénine pour l'administration transdermique de diosgénine à un patient pour traiter un diabète de type II chez le patient, dans lequel le procédé comprend les faits :
- de préparer une phase huileuse comprenant une combinaison 1 : 1 d'huile de sésame et d'huile de graines de courge ;
- d'ajouter 15 % d'émulsifiants sous la forme d'un mélange de 1 : 1 de surfactant non ionique et d'un co-surfactant à 60 % de la phase huileuse ;
- de dissoudre 40 mg/kg de diosgénine dans la phase huileuse à température ambiante en utilisant un agitateur magnétique ; et
- d'ajouter de l'eau distillée en goutte à goutte au mélange tout en agitant par l'intermédiaire de l'agitateur magnétique pour obtenir une nano-émulsion huile-en-eau avec un rapport huile-eau de 4 : 1.
